# EUROPÄISCHE PATENTSCHRIFT

(11) **EP 3 171 348 B1**
(45) Veröffentlichungstag und Bekanntmachung des Hinweises auf die Patenterteilung: **23.12.2020**
(21) Anmeldenummer: 16199781.2
(22) Anmeldetag: 21.11.2016
(51) Int. Cl.: G08B 21/04, A61B 5/01, A61B 5/11, G08B 5/36, G08B 25/08, G08B 25/00

(54) **VORRICHTUNG UND VERFAHREN ZUM BESTIMMEN EINER ATYPISCHEN BEWEGUNG**
DEVICE AND METHOD FOR DETERMINING AN ATYPICAL MOVEMENT
DISPOSITIF ET PROCÉDÉ DE DÉTERMINATION D'UN MOUVEMENT ATYPIQUE

(30) Priorität: 19.11.2015 DE 102015120055
(43) Veröffentlichungstag der Anmeldung: 24.05.2017
(73) Patentinhaber: pikkerton GmbH, 13629 Berlin (DE)
(72) Erfinder: FEIGE, Lothar, 13437 Berlin (DE)
(74) Vertreter: Bittner, Thomas L.

(56) Entgegenhaltungen:
- GB-A- 2 498 951
- US-A1- 2015 096 352
- US-A1- 2015 323 388

## Beschreibung

Die Erfindung betrifft eine Vorrichtung und ein Verfahren zum Bestimmen einer atypischen Bewegung, insbesondere Sturzerkennung.

### Hintergrund

Derartige Technologien dienen dem Erkennen von atypischen Bewegungsmustem, insbesondre eines Sturzes einer Person. Ein anderes Beispiel für eine atypische Bewegung ist das schnelle Herabsinken einer Person in ein Sitzmöbel, was eine Gefährdungssituation anzeigen kann, zum Beispiel das Auftreten einer Herzschwäche.

Zum Bestimmen atypischer Bewegungen, insbesondere zur Sturzerkennung, wurden verschiedene Verfahren vorgeschlagen. Hierzu zählt die Verwendung einer räumlichen Anordnung von mehreren Sensoren zur Raumüberwachung. Aktive Sensoren senden hierbei Signale aus, die von Reflektoren am Körper einer Person reflektiert werden. Auch das Auswerten von bildgebenden Verfahren zur Sturzbestimmung wurde vorgesehen. Mit diesem Verfahren ist eine aufwendige Installation der benötigten Detektoreinrichtung verbunden.

Bildgebende Verfahren sind in diesem Bereich inakzeptabel, da die Integration dieser Technologie massiv die Privatsphäre der zu überwachenden Personen verletzt. Es werden hochaufgelöste Bilder erfasst, gespeichert und wahlweise sogar zur externen Bewertung weitergeleitet. Im Fall von Sicherheitslücken in der IT-Infrastruktur gelangen Bilder / Videoaufnahmen aus der Wohnung an Dritte.

Verfahren auf Basis von ToF ("Time of Flight")-Kameras sind nicht nur bildgebend, sondern auch sehr teuer. Auch Verfahren wie Radarauswertung sind kostspielig.

Weiterhin wurde die Verwendung sogenannter Tags am Körper der zu überwachenden Person vorgeschlagen. Tags funktionieren mit unterschiedlichen Sensoren, teilweise wird der Luftdruck ausgewertet, der in Bodennähe natürlich zunimmt. Andere Sensoren werten mit Hilfe von xyz-Beschleunigungssensoren Beschleunigungen und Bewegungen aus und analysieren diese Daten im Hinblick auf ein Sturzrisiko. Andere Sensoren ermöglichen mit anderen Verfahren eine genaue Ortung, die dann wiederum auch einer Bewegungs- oder Sturzanalyse zugeführt wird. Nachteile dieser Tags / mobilen Sensoren sind: Unkomfortable Trageigenschaften, zum Beispiel während der Schlafphase; Verrutschen der Tags am Körper, was die Daten unbrauchbar machen kann; ungewolltes Ablegen; anderweitige Beeinträchtigung, zum Beispiel in der Badewanne; batteriebetriebene Ausführung, was ständige Wartungs- / Serviceintervalle erfordert.

Es existieren weiterhin aktive Verfahren, bei denen Radarstrahlung, IR-Strahlung oder andere Formen von Energien in den Raum auf die Person gerichtet werden. Auch dies führt zu Akzeptanzproblemen bei den Nutzem.

Aus dem Dokument WO 2015 / 171365 A1 ist eine Hilfs- oder Unterstützungsvorrichtung für eine Person bekannt, mit der eine Lageüberwachung ausgeführt wird. Die Hilfs- oder Unterstützungsvorrichtung weist eine Sitz- oder Liegefläche auf, beispielsweise bei einem Krankenbett oder einem Rollstuhl. Mit Hilfe von Sensoren, die ein Thermopile-Array aufweisen können, wird überwacht, ob sich auf der Sitz- oder Liegefläche eine Person befindet. In einer Ausführungsform sind an einem Bett mehrere Sensoren vorgesehen, die den Bereich auf der Liegefläche sowie den Bereich unterhalb des Betts überwachen, indem Thermobilder ausgewertet werden. Wird ein Wärmemuster oberhalb eines Abstands vom Boden, auf dem das Bett steht, detektiert, ist vorgesehen, eine Bewegung des Wärmemusters zu überwachen und zu verfolgen, um zu bestimmen, ob das Wärmemuster für wenigstens eine begrenzte Zeit sich unterhalb der Höhe über dem Fußboden bewegt. Wenn dies der Fall ist, wird das als ein Fallen der Person aus dem Bett bestimmt.

Das Dokument US 2015 / 0096352 A1 betrifft ein Verfahren zum Bestimmen einer oder mehrerer Quellen von Kohlenmonoxid in Verbindung mit einer sogenannten Smart-Home-Anwendung.

Im Dokument GB 2498951 A ist eine nicht-invasive Überwachungsvorrichtung zum Detektieren der Bewegung einer Person innerhalb ihres Wohnraums beschrieben, wobei die Überwachungsvorrichtung dreidimensionale thermische Sensoren in Form eines Thermopile-Arrays aufweist.

Das Dokument US 2015 / 0029320 A1 beschäftigt sich mit einer bildgebenden Vorrichtung, welche ein Gehäuse und eine im Gehäuse aufgenommene Kamera aufweist, mit der kontinuierlich Bilder detektiert werden. Die Kamera ist eine Thermobild-Kamera.

Aus dem Dokument US 2009 / 0242769 A1 ist ein Detektor für Schwarzkörper-Strahlung eines Menschen bekannt.

### Zusammenfassung

Aufgabe der Erfindung ist es, eine Vorrichtung und ein Verfahren zum Bestimmen einer atypischen Bewegung einer Person anzugeben, insbesondere zur Sturzerkennung, die auf einfache und effiziente Art und Weise das Bestimmen atypischer Bewegungen von Personen ermöglichen, insbesondere im Wohnraumumfeld.

Zur Lösung sind eine Vorrichtung sowie ein Verfahren zum Bestimmen einer atypischen Bewegung einer Person nach den unabhängigen Ansprüchen 1 und 11 geschaffen. Ausgestaltungen sind Gegenstand von abhängigen Unteransprüchen.

Als atypische Bewegung der Person kann zum Beispiel ein Sturz bestimmt werden. Aber auch ein plötzliches und schnelles Absinken einer Person aus dem Stand in ein Sitzmöbel oder auf ein Bett kann eine atypische Bewegung sein, die mit Hilfe der vorgeschlagenen Technologien als Gefahrensituation erkannt werden kann.

Im Bereich der Sichtseite können mehrere Thermopile-Arrays zum Detektieren der IR-Strahlung für eine oder mehrere Personen vorgesehen sein.

Die Vorrichtung kann eingerichtet sein, den der Sichtseite vorgelagerten Messraum zu erfassen, also frei von weiteren IR-Sensoren, beispielsweise einen gesamten Wohnraum (Zimmer).

Eine Nachprüfeinrichtung kann in dem Gehäuse vorgesehen sein, die an die Auswerteeinrichtung koppelt und eingerichtet ist, als Reaktion auf ein vorläufiges Erkennen der atypischen Bewegung beim Prozess zum Bestimmen der atypischen Bewegung eine Nutzereingabe von der detektierten Person zu detektieren. Das Vorsehen der Nachprüfeinrichtung ermöglicht das Ausführen unterschiedlicher Betriebsarten. So kann vorgesehen sein, vor dem Bereitstellen des das Bestimmen der atypischen Bewegung anzeigenden Signals am Datenausgang der Auswerteeinrichtung die Nutzereingabe zu detektieren oder alternativ das Signal am Datenausgang der Auswerteeinrichtung bereitzustellen, wenn innerhalb eines vorbestimmten Zeitraums keine Nutzereingabe mit Hilfe der Nachprüfeinrichtung detektiert wird. Mit Hilfe der Nachprüfeinrichtung kann die Wahrscheinlichkeit für eine Fehlbestimmung der atypischen Bewegung gemindert werden. Die Nutzung der Nachprüfeinrichtung ergänzt den Auswerteprozess der von der passiven IR-Detektionseinrichtung bereitgestellten Detektorsignale.

Die Nachprüfeinrichtung kann eingerichtet sein, ein optisches Nutzersignal, ein akustisches Nutzersignal und / oder ein Nutzerberührungssignal zu detektieren. Ein akustisches Nutzersignal, zum Beispiel eine Sprachnachricht, kann mit Hilfe einer Mikrofoneinrichtung detektiert werden. Ein optisches Nutzersignal kann zum Beispiel in Verbindung mit einer Lichtschranke erfasst werden. Alternativ oder ergänzend kann mittels der passiven IR-Detektionseinrichtung eine Geste erfasst werden. Die passive IR-Detektionseinrichtung erfüllt dann eine Funktionalität der Nachprüfeinrichtung. Ein Nutzerberührungssignal kann mit Hilfe einer berührungssensitiven Detektorfläche oder eines Tasters erfasst werden. Das Nutzersignal kann von der Auswerteeinrichtung als Information gewertet werden, die anzeigt, dass das vorläufige Erkennen der atypischen Bewegung, insbesondere eines Sturzes, nicht zutreffend ist, weil sich die detektierte Person mit dem Nutzersignal zurückmeldet. Alternativ kann das Nutzersignal als eine Bestätigung der vorläufig erkannten atypischen Bewegung ausgewertet werden, um im Anschluss das das Bestimmen der atypischen Bewegung anzeigende Signal am Datenausgang der Auswerteeinrichtung bereitzustellen.

Die Mikrofoneinrichtung kann eingerichtet sein, zum Beispiel verdächtige mit dem Sturz in Verbindung stehende Geräusche zu detektieren, beispielweise Klopfgeräusche, Fallgeräusche, Hilferufe und / oder Schreie. Für das Bestimmen der Geräusche nutzbare (softwarebasierte) Verfahren sind Stand der Technik als solche bekannte ("Sound Classification") und können bereits in einem Halbleiterchips bereits integriert sein.

Es können ein oder mehrere Mikrophone vorgesehen sein. Hierbei kann vorgesehen sein, die Richtung zu erfassen, aus der Geräusche, vermeintliche Hilferufe und / oder Sturzgeräusche kommen. Die detektierten Signale können zu einer Anomalieerkennungsanalyse genutzt werden. Hierzu werden als solche bekannte Verfahren verwendet (zum Beispiel: Abweichungen vom Mittelwert und erlernten / errechneten Mittelwerten) und über Scorecard-Verfahren miteinander kombiniert. Bei Überschreitung eines bestimmten Schwellwertes wird dann ein Alarm ausgelöst.

Es kann ein externer Rauchmelder vorgesehen sein, welcher eingerichtet ist, für Rauchmelder typische Frequenzen und Puls-/ Pausenzeiten zu erkennen.

Bei bekanntem Aufenthaltsort der hilfebedürftigen Person, welcher aus der Analyse von Thermopile-Sensoren bestimmt werden kann, kann vorgesehen sein, eine resultierende Empfangscharakteristik der Mikrophone schmaler zu gestalteten. Ein Mikrophon hat üblicherweise eine relativ breite Empfangskeule (Empfangscharakteristik) - möglichst viele Geräusche sollen aus allen möglichen Richtungen aufgenommen werden. Im Alarmfall kann diese Empfangskeule mittels Nutzung mehrerer Mikrophone und digitaler Filter, die zum Beispiel in DSP-Bausteinen realisiert werden können, schmaler gestaltet werden. Hierdurch wird die Empfangskeule länger und kann so auf die Person gerichtet werden. So können Störgeräusche wie Fernseher ausgeblendet werden, und die akustische Kommunikationsqualität kann verbessert werden. Dieses Vorgehen kann als Reaktion auf die vorherige optische Analyse ausgeführt werde, aus der bestimmt werden kann, an welcher Stelle die Person liegt.

Hierbei kann das als solche bekannte Beamforming eingesetzt werden. Eine resultierende Empfangscharakteristik wird auf die Person gerichtet, um akustische Störsignale zu unterdrücken, zum Beispiel laute Geräusche von einem Fernseher. Hierdurch kann die eventuelle Störung eines Sprach- oder Telefonanrufs nach dem Erkennen der atypischen Bewegung gemindert werden.

Die Information einer Falschinterpretation einer Situation kann in Verbindung mit der getätigten Rückmeldung des Benutzers verwendet werden, den Erkennungsprozess im Hinblick auf seine Konfiguration permanent zu verbessern und so zukünftig die Falschmeldungen weiter zu minimieren. Es können hierbei Entscheidungsschwellen adjustiert werden. Ein Klassifikator für die Erkennung einer atypischen Situation oder eines Sturzes kann unschärfer gestellt werden. Ein Entscheidungsverfahren kann aufgrund dieser Rückmeldung trainiert und somit kontinuierlich verbessert werden.

Eine Signalisierungseinrichtung kann in dem Gehäuse vorgesehen sein, die an die Auswerteeinrichtung koppelt und eingerichtet ist, auf das vorläufige Erkennen der atypischen Bewegung durch die Auswerteeinrichtung ein optisches und / oder ein akustisches Signal auszugeben. Ein akustisches Signal kann beispielsweise mit Hilfe einer Lautsprechereinrichtung oder eines Buzzers als eine Ansage an die potentiell detektierte Person ausgegeben werden. Ein optisches Signal kann beispielsweise als ein Blinksignal ausgegeben werden. Hierzu kann beispielsweise eine lichtemittierende Diode in Verbindung mit der Nachprüfeinrichtung vorgesehen sein. Die Ausgabe der Signalisierungseinrichtung kann dem Nutzer anzeigen, dass eine atypische Bewegung vorläufig durch die Auswerteeinrichtung erkannt wurde und eine Nachprüfung dieser vorläufigen Bewegungserkennung stattfindet. Eine hierauf erfolgende Nutzereingabe kann das Ergebnis der vorläufigen Bewegungserkennung bestätigen oder verwerfen.

Es kann eine Bewegungsdetektoreinrichtung in dem Gehäuse vorgesehen sein, die an Auswerteeinrichtung koppelt und eingerichtet ist, beim Prozess zum Bestimmen der atypischen Bewegung Bewegungseigenschaften für die detektierte Person zu bestimmen. Mit Hilfe der Bewegungsdetektoreinrichtung können als Bewegungseigenschaften ein Bewegen nach unten, beispielsweise in y-Richtung, ein nicht-Bewegen und / oder eine beschleunigte Bewegung detektiert werden. Hierbei kann vorgesehen sein, aus den Detektorsignalen der IR-Detektionseinrichtung zunächst einen räumlichen Bezugspunkt für die detektierte Person zu bestimmen, um dann für diesen Bezugspunkt ergänzende Bewegungseigenschaften zu erfassen. Alternativ oder ergänzend können ein oder mehrere Stereomikrofone eingesetzt werden.

Es kann eine elektronische Filtereinrichtung in dem Gehäuse, die zumindest an IR-Detektionseinrichtung koppelt und eingerichtet ist, vorgesehen sein, die von der passiven IR-Detektionseinrichtung bereitgestellten Detektorsignale zu filtern. Mit Hilfe der elektronischen Filterung kann zum Beispiel eine Verbesserung des Signal-Rausch-Verhältnisses für die Detektorsignale erzielt werden. Die elektronische Filtereinrichtung kann einen Tiefpass-Filter aufweisen.

Eine Kommunikationseinrichtung kann in dem Gehäuse vorgesehen sein, die an die Auswerteeinrichtung koppelt und eingerichtet ist, als Reaktion auf das Erkennen der atypischen Bewegung ein Kommunikationssignal abzusetzen. Das Kommunikationssignal kann dazu benutzt werden, eine Telekommunikationsverbindung aufzubauen, beispielsweise zu einem Festnetzanschluss und / oder einem Mobilfunktelefon, welches im vorgelagerten Messraum oder in dessen Nähe angeordnet ist. Wenn die Vorrichtung zum Bestimmen der atypischen Bewegung mit einem Mikrofon sowie einem Lautsprecher ausgestattet ist, können diese Einrichtungen in diesem Zusammenhang als Freisprecheinrichtung zum Einsatz kommen. Zum Ausbilden der Kommunikationsverbindung kann die Kommunikationseinrichtung ein oder mehrere Module aus der folgenden Gruppe aufweisen: Bluetooth-Modul, WLAN-Modul, GSM-Modul, 3G-Modul und LTE-Modul. Die Vorrichtung kann (zum Beispiel über Bluetooth oder WLAN) in der Nähe befindliche Mobiltelefone nutzen, um zum Zweck des Aufbaus einer Sprachverbindung eine Nummer zu wählen oder eine SMS / E-Mail zu verschicken. Die Vorrichtung kann "selbst" durch WLAN, GSM, LTE oder dergleichen einen Sprach-Call aufbauen und / oder SMS / E-Mail verschicken (oder anderweitige Benachrichtigungen).

Eine Notfallsignaleinrichtung kann in dem Gehäuse vorgesehen sein, die an die Auswerteeinrichtung koppelt und eingerichtet ist, als Reaktion auf das Erkennen der atypischen Bewegung ein Notfallsignal abzusetzen. Die Notfallsignaleinrichtung kann mit der Kommunikationseinrichtung zusammenwirken, derart, dass das Notfallsignal als Notfallnachricht über die mittels der Kommunikationseinrichtung aufgebaute Kommunikationsverbindung übertragen wird. Alternativ oder ergänzend kann ein optisches und / oder ein akustisches Notfallsignal über eine entsprechende Ausgabeeinrichtung abgesetzt werden, beispielsweise eine lichtemittierende Diode oder die Lautsprechereinrichtung. Zum Übertragen des Notfallsignals kann die PLC-Technik genutzt werden (PLC-Powcrline Communication). Auf diese Weise können Datensignale an einen beliebigen Empfänger übermittelt werden, beispielsweise eine entfernte Serverstation.

Auch ein Relais kann als Notsignal verwendet werden. Falls mit der Vorrichtung kein Lichtschalter ersetzt wird, sondern eine "Standalone"-Implementierung vorgesehen ist, können am Relais auch Notsignale angeschlossen werden, zum Beispiel ein rotes Warnlicht im Krankenhausflur und / oder ein akustischer Signalgeber.

Das Gehäuse kann unter Verwendung einer Unterputz- oder einer Aufputz-Schalterdose gebildet sein. Die Sichtseite kann im Bereich einer Abdeck- oder Frontplatte der Schalterdose angeordnet sein.

Die Sichtseite kann auf einer Schalterfrontplatte gebildet sein. Die Schalterfrontplatte kann die Frontplatte eines Lichtschalters sein, der als Kippschalter oder Taster ausgeführt sein kann.

Es kann eine Anschlusseinrichtung in dem Gehäuse vorgesehen sein, die an die passive IR-Detektionseinrichtung und die Auswerteeinrichtung koppelt und mit einem Spannungsversorgungsnetz verbunden und eingerichtet ist, die passive IR-Detektionseinrichtung und die Auswerteeinrichtung mit einer jeweiligen Betriebsspannung zu versorgen. Das Spannungsversorgungsnetz kann ein 230 V-Versorgungsnetz sein. Insbesondere beim Integrieren der Vorrichtung zum Bestimmen der atypischen Bewegung der Person in eine Schalterdose ist ein einfacher Anschluss an das Spannungsversorgungsnetz ermöglicht, da dieses in der Schalterdose selbst regelmäßig zur Verfügung steht.

In Verbindung mit dem Verfahren zum Bestimmen einer atypischen Bewegung einer Person, insbesondere zur Sturzerkennung, können die vorangehend in Verbindung mit der Vorrichtung erläuterten Ausgestaltungen entsprechend vorgesehen sein. Die verschiedenen Einrichtungen und Komponenten der Vorrichtung ermöglichen entsprechende Ausgestaltungen des Verfahrens in beliebiger Kombination.

Die Vorrichtung kann einen Temperatursensor aufweisen, der an die Auswerteeinrichtung koppelt. Auf diese Weise kann zum Beispiel eine Raumtemperatur in dem vorgelagerten Messraum erfasst werden. Die Temperatursensorsignale können beim Bestimmen der atypischen Bewegung als ergänzendes Entscheidungskriterium herangezogen werden. Auf diese Weise ist es beispielweise möglich, kritische Situation wie im Winter offenstehende Fenster zu detektieren und gesundheitliche Schäden vorzubeugen.

Die Vorrichtung kann auf der Sichtseite ein lichtemittierendes Element aufweisen, mit dem ein Orientierungslicht in den Messraum hinein abgegeben wird. Beispielsweise kann so bei Dunkelheit ein Orientierungsspot im Messraum bereitgestellt werden, zum Beispiel auf dem Fußboden.

Die Vorrichtung kann einen Luftfeuchtigkeits- und / oder einen VOC- (Luftgütemessung) Sensor aufweisen, der an die Auswerteeinrichtung koppelt. Hierdurch ist es ermöglicht, gesundheitsschädliche klimatische Umgebungen zu detektieren, zum Beispiel Schimmelbildung und / oder mangelnde Lüftung und auch die damit verbundene scheinbare Inaktivität des Bewohners.

Die Vorrichtung kann einen Helligkeitssensor aufweisen, der an die Auswerteeinrichtung koppelt. Die Helligkeit der Betriebs-LED. Die Betriebs-LED zeigt an, ob das Gerät ordentlich arbeitet (Sensoren o.k.?) und / oder eine funktionstüchtige Alarmierungsstrecke besteht, zum Beispiel ob eine Verbindung zum GSM-Netz oder WiFi besteht. Grün kann bedeuten o.k., rot heißt Fehler. Damit diese LED tagsüber sichtbar ist und nachts nicht stört, kann sie im Dunkeln etwas heruntergedimmt werden. Das Dimmen kann in Abhängigkeit von Umgebungs-Helligkeitssignalen gesteuert werden, die mittels des Helligkeitssensors erfasst werden. Zum Beispiel kann im Dunkeln nach dem Bestimmen der atypischen Bewegung die Betriebs-LED angeschaltet oder im bereits angeschalteten Zustand heller geregelt werden, um ein Orientierungslichtsignal zur Sturzvermeidung bereitzustellen.

Es kann am Gerät, insbesondere im Gehäuse aufgenommen, eine weiße LED vorgesehen sein, zum Beispiel auf der Unterseite, die den Fußboden etwas beleuchtet, zum Beispiel wenn es dunkel ist und / oder wenn Bewegung erkannt wird.

Der Thermopile-Array mit der zugeordneten Auswerteinrichtung können ergänzend eingerichtet sein, entstehende Brände zum Beispiel in einer Küche zu bestimmen. Hierfür kann das Thermopile-Array direkt die Temperaturen liefern, die im Falle eines entstehenden Brandes entsprechend hoch sind. Ansteigende Temperaturen und eine flächenmäßige Ausbreitung heißer Stellen sind ein hinreichendes Merkmal dafür.

Mögliche Funkschnittstellen zur Signalisierung sind: Mobilfunk 2G, 3G, 4G, 5G, Narrowband-IoT, ZigBee, Zwave, 868 MHz und oder lizenzfreie ISM-Bänder wie 433, 868, 900, 915, 2.4 GHz. Dies sind die Funkschnittstellen, die üblicherweise für eine Signalisierung / Übermittlung einer Alarmmeldung verwendet werden oder aber für eine wohnungsinterne Kommunikation mehrerer Geräte untereinander.

Als Protokolle können bei der Signalisierung ein oder mehrere der folgenden Signalübertragungsprotokolle zum Einsatz kommen: Thread, 6LowPAN, IP (Internet Protokoll), Email / SMTP ("Simple Mail Transfer Protocol"), FTP ("File Transfer Protocol"), MQTT ("Message Queue Telemetry Transport"), SNMP ("Simple Network Management Protocol"), SMS ("Short Message Service"), Sprachanruf GSM, Sprachanruf über LTE und / oder Sprachanruf über IP.

Es kann ein Schalten einer elektrischen Last vorgesehen sein, zum Beispiel einer Deckenleuchte. Im Gehäuse können einen Taster und ein Relais vorgesehen sein. In einer Wohnung kann das Relais nach Tasterbetätigung die Deckelleuchte an und aus steuern. Es sind auch Szenarien denkbar, zum Beispiel im Krankenhaus, in denen das Relais eine separate rote Warmleuchte, beispielweise auf dem Krankenhausflur, ansteuert, um den Alarmfall optisch zu signalisieren. In diesem Fall könnte das Deckenlicht natürlich nicht mehr angesteuert werden.

Es können unterschiedliche Fälle / Funktionen unterschieden werden.

Es kann eine direkte Sturzerkennung mittels Messwerterfassung vorgesehen sein, ggfs. Interpolation, Segmentierung, Extrahierung von Merkmalen, Klassifizierung.

Alternativ kann eine Gefahrenerkennung durch sonstige integrierte Sensoren (Temperatur, VOC, etc.) erfolgen, zum Beispiel Branderkennung und / oder kalte Temperaturen, Schimmelbildung, ungesundes Raumklima, etc.

Nach einer weiteren Alternative, wahlweise in Ergänzung, kann eine Inaktivitätserkennung vorgesehen sein (Abweichen vom "Normal"). Die Vorrichtung lernt das "nomiale" Verhalten des Bewohners, d.h. übliche Zeiten, Bewegungen, Auswerten von Raumtemperaturen und - helligkeiten, Lichtschaltverhalten und / oder Lüftungsverhalten, etc. Einzelne oder mehrere Parameter, die das normale Verhalten kennzeichnen werden, der "Anomalieerkennung" zugeführt. Dies können statistische Ansätze (Abweichungen von Mittelwerten, Histogramm-Analyse) sein. Über ein Scorecard-Verfahren kann dann entschieden / festgelegt werden, wann die Vorrichtung einen außergwöhnlichen Zustand erkennt, woraus auf eine atypische Bewegung geschlossen werden kann.

Die Vorrichtung zum Bestimmen der atypischen Bewegung kann eingerichtet sein, ein normales manuelles Schaltverhalten der Person für eine atypische Bewegung erkannt werden soll, zu bestimmen. Dies kann beim Bestimmen der atypischen Bewegung herangezogen werden. Das manuelle Schaltverhalten kann Folgendes betreffen: Abspeichern in einer Tabelle, Uhrzeit und Schaltvorgang (an/aus).

Eine Konfiguration der Vorrichtung zum Bestimmen der atypischen Bewegung kann eine oder mehrere drahtlos-Schnittstellen vorsehen. Zur Konfiguration können ein oder mehrere der folgenden Konfigurationsmerkmale vorgesehen sein: Alarm-Ziele (Telefonnummern für SMS-Versand, Telefonnummern für den Aufbau von Sprachanrufen, auch mehrere nacheinander, Email-Adresse für eine Email-Signalisierung, Parameter für sonstige Signalisierungsformen und -wege wie zum Beispiel Server-Adressen, Ordnernamen für FTP-Server, Usernamen und Passwörter).

Mehrere Geräte in einer Wohnung können über drahtlose Schnittstellen miteinander kommunizieren und Sensorinformationen austauschen. Dafür kann eine Master-Slave-Struktur im Funknetzwerk gebildet sein, dahingehend, dass ein Master Auswertungen vornimmt, die nur mit Sensordaten durchgeführt werden können, die auch aus anderen Geräten stammen, wie zum Beispiel Inaktivität in allen Räumen mit Sensoren, zum Beispiel allen Räumen eines Hauses. Wenn die Oma sich im Schlafzimmer bewegt und aufhält, ist das sicherlich erstmal in Ordnung und kann nicht als Inaktivität in Hinblick auf die Gesamtwohnung detektiert werden. Die Vorrichtung zum Bestimmen der atypischen Bewegung kann weitere Sicherheitsmerkmale aufweisen, um Angriffe zu vermeiden. So können über die Drahtlosschnittstelle(n) mechanische Interaktionen verlangt und dann eine entsprechende Benutzereingabe erfasst werden, um den Konfigurationsmodus.

Die Vorrichtung kann über einen integrierten Webserver verfügen, den man per normalen Browser (Smartphone, Notebook, PC) bedienen kann. Über diesen kann das Gerät konfiguriert werden: Alarmquellen, Alarmziele und / oder verschiedene Einstellungen (Netzwerk) Features (Lichtsteuerung, etc.). Dieser Konfigurationsmodus kann gegen externe Angreifer geschützt sein. Die Ausführung einer TAP-Eingabe, zum Beispiel ein Klopfen auf den TAP-Sensor oder Betätigung eines Tasters, zielt im Wesentlichen darauf ab, dass das Gerät vom konfigurierenden Benutzer eine bestimmte Klopf- oder Tastereingabe verlangt, um sicherzustellen, dass dieser auch in der Wohnung ist und das Gerät tatsächlich konfiguriert. Damit können externe Hacker oder unfreundliche Personen außerhalb der Wohnung von der Konfigurationsmöglichkeit explizit und sehr sicher ausgeschlossen werden.

Es können genetische Ansätze genutzt werden: Untersuchung von zufällig gewählten / computergenerierten Merkmalen und Überprüfung dieser Merkmale in Hinblick auf ihre Tauglichkeit / Fitness. Dazu werden üblicherweise qualifizierte Testdaten ("Sturz" / "kein Sturz") zur Bewertung der Merkmale herangezogen. Die Merkmale werden mutiert, erneut bewertet und führen so iterativ zu tauglichen Merkmalen.

Es kann vorgesehen sein, bei der Auswertung der erfassten Sensorsignale neuronale Netze einzusetzen, Verfahren wie bei (b), Training der neuronalen Netze mit vorqualifizierten Testdaten.

Es kann vorgesehen sein, bei der Auswertung statistischer Ansätze zu verwenden. Normale oder übliche Verhaltensweisen oder Bewegungsmuster werden für die Person anhand der erfassten Sensorsignale aufgezeichnet (Leave Trace) und als Normalfall gespeichert, zum Beispiel Bewegungen zwischen einem Fernsehsessel und einer Eingangstür. Hiervon abweichende Bewegungsmustern werden beim Auswerten mittels der Auswerteeinrichtung als anomal / meldewürdig (atypisch) qualifiziert. Hierbei lernt die Vorrichtung das Normalmuster.

Es kann vorgesehen sein, bei der Auswertung eine Aktivitätsanalyse in der Wohnung zu verwenden. Dies kann mittels Auswerten der Signale des Thermopile-Arrays ausgeführt werden. Alternativ oder ergänzend kann die Verwendung eines oder mehrerer PIR-Elemente (reiner Bewegungssensor) vorgesehen sein, für die die erfassten Signale zur Aktivitätsanalyse ausgewertet werden. Mehrere Sensorelemente können über eine ganze Wohnung verteilt sein, um die Aktivitätsanalyse auf die gesamte Wohnung auszudehnen.

Die verschiedenen Schritte zum Auswerten oder Analysen können mittels der funktionalen Komponenten der Vorrichtung zum Bestimmen der atypischen Bewegung und / oder Datenverarbeitungseinrichtungen außerhalb der Vorrichtung ausgeführt werden, zum Beispiel im Rahmen eines Cloud-Computings.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische Blockdarstellung einer Vorrichtung zum Bestimmen einer atypischen Bewegung einer Person,
- Fig. 2: eine schematische Blockbilddarstellung zum Erläutern eines Verfahrens zum Bestimmen einer atypischen Bewegung einer Person,
- Fig. 3: eine schematische Darstellung von zwei Matrixanordnungen von Sensorpixeln für Thermopile-Arrays,
- Fig. 4: eine schematische Darstellung von Interpolationsdaten,
- Fig. 5: eine schematische Darstellung für zwei erkannte Objekte und
- Fig. 6: eine schematische Darstellung in Verbindung mit der Filterung der IR-Messsignale vor (linke Seite) sowie nach einer Rauschunterdrückung (rechte Seite).

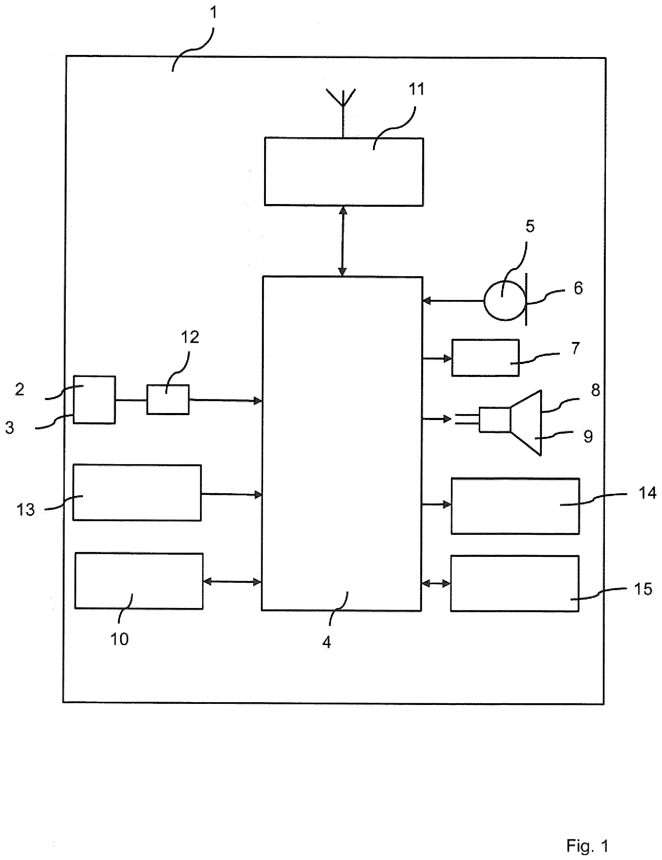

Fig. 1 zeigt eine schematische Darstellung für eine Vorrichtung zum Bestimmen einer atypischen Bewegung einer Person, insbesondere zum Bestimmen eines Sturzes einer Person (Sturzerkennung). In einem Gehäuse 1 ist eine passive IR-Detektionseinrichtung 2 (IR - Infrarot) angeordnet, die wenigstens ein Thermopile-Array 3 aufweist. Mit Hilfe des Thermopile-Arrays 3 können in einem überwachten Messraum, welcher dem Gehäuse 1 mit dem Thermopile-Array 3 vorgelagert ist, IR-Strahlungssignale für ein oder mehrere Personen detektiert werden. Gemäß Fig. 1 koppelt an die IR-Detektionseinrichtung 2 eine Auswerteeinrichtung 4, die insbesondere einen Prozessor umfasst.

Bei der dargestellten Ausführungsform ist die Auswerteeinrichtung 4 weiterhin verbunden mit einer Nachprüfeinrichtung 5, die in der gezeigten Ausführung ein Mikrofon 6 sowie einen Taster 7 aufweist, zum Beispiel einen MEMS-TAP-Detektor (Erschütterungs- bzw. Beschleunigungssensor).

Weiterhin ist eine Signalisierungseinrichtung 8 vorgesehen, die insbesondere mit einem Lautsprecher 9 und / oder einem Buzzer gebildet ist.

Eine Kommunikationseinrichtung 10 koppelt an die Auswerteeinrichtung 4 und ist eingerichtet, als Reaktion auf das Erkennen einer atypischen Bewegung ein Kommunikationssignal abzusetzen. Das Kommunikationssignal kann dazu benutzt werden, eine Telekommunikationsverbindung aufzubauen, beispielsweise zu einem Festnetzanschluss und / oder einem Mobilfunktelefon, welches im vorgelagerten Messraum oder in dessen Nähe angeordnet ist. Das Mikrofon 6 sowie der Lautsprecher 9 können in diesem Zusammenhang als Freisprecheinrichtung zum Einsatz kommen. Zum Ausbilden der Kommunikationsverbindung kann die Kommunikationseinrichtung 10 mit einem oder mehreren Modulen 11 aus der folgenden Gruppe zusammenwirken: Bluetooth-Modul, WLAN-Modul, GSM-Modul, 3G-Modul und LTE-Modul.

Dem Thermopile-Array 3 ist bei der gezeigten Ausführung eine Filtereinrichtung 12 nachgelagert, mit der die empfangenen Messsignale elektrisch gefiltert werden, insbesondere zum Verbessern des Signal-Rausch-Verhältnisses. Alternativ oder ergänzend kann eine Signalfilterung in der Auswerteeinrichtung 4 ausgeführt werden. Die Filterung, wie alternativ oder ergänzend weitere der hier beschriebenen Funktionalitäten der Vorrichtung, kann mittels geeigneter Hardware- und / oder Softwarekomponenten durchgeführt werden.

Mittels einer Bewegungsdetektoreinrichtung 13 kann eine Bewegungsinformation für Objekte im Messraum erfasst werden, zum Beispiel mittels eines Beschleunigungsdetektors.

Eine Notfallsignaleinrichtung 14 koppelt an die Auswerteeinrichtung 4 und kann eingerichtet sein, als Reaktion auf das Erkennen der atypischen Bewegung ein Notfallsignal abzusetzen. Die Notfallsignaleinrichtung 14 kann mit der Kommunikationseinrichtung 10 zusammenwirken, derart, dass das Notfallsignal als Notfallnachricht über die mittels der Kommunikationseinrichtung 10 aufgebaute Kommunikationsverbindung übertragen wird. Alternativ oder ergänzend kann ein optisches und / oder ein akustisches Noten fall signal über eine entsprechende Ausgabeeinrichtung abgesetzt werden, beispielsweise eine lichtemittierende Diode oder den Lautsprecher 9. Zum Übertragen des Notfallsignals kann die PLC-Technik genutzt werden (PLC-Powerline Communication). Auf diese Weise können Datensignale an einen beliebigen Empfänger übermittelt werden, beispielsweise eine entfernte Serverstation.

Die Auswerteeinrichtung 4 steht weiterhin in Verbindung mit einer Anschlusseinrichtung 15, über die ein Anschluss an ein 230V-Versorgungsnetzwerk bereitgestellt ist.

Die Vorrichtung kann als Einbaugerät für Elektroinstallations-Schalterdosen ausgeführt sein. Es kann zum Beispiel in einer Unterputz- oder in einer Aufputz-Schalterdose installiert werden. Mittels optionaler Verwendung eines 230V-Relais kann beispielsweise ein vorhandener Lichtschalter leicht und ohne Funktionseinbußen ersetzt werden. Die Lichtsteuerung über das Relais kann zum Beispiel wie folgt realisiert werden: Mittels eines Tasters in der Frontplatte; mittels erkannter Gesten (Gestensteuerung); mittels erkannter akustischer Signale (Spracherkennung / -steuerung); mittels Tipp- / Berührungsvorgänge an der Frontplatte in Verbindung mit einem integrierten Beschleunigungssensor; und / oder mittels eines Smartphones oder eines Web-Browsers.

Das Verhalten der Vorrichtung im Betrieb kann mittels Anpassen oder Einstellen der Steuerungs-Software, insbesondere bezüglich des Betriebs der Auswerteeinrichtung 4, über eine Schnittstelle konfiguriert werden, beispielsweise über Bluetooth- oder WLAN-Kommunikation.

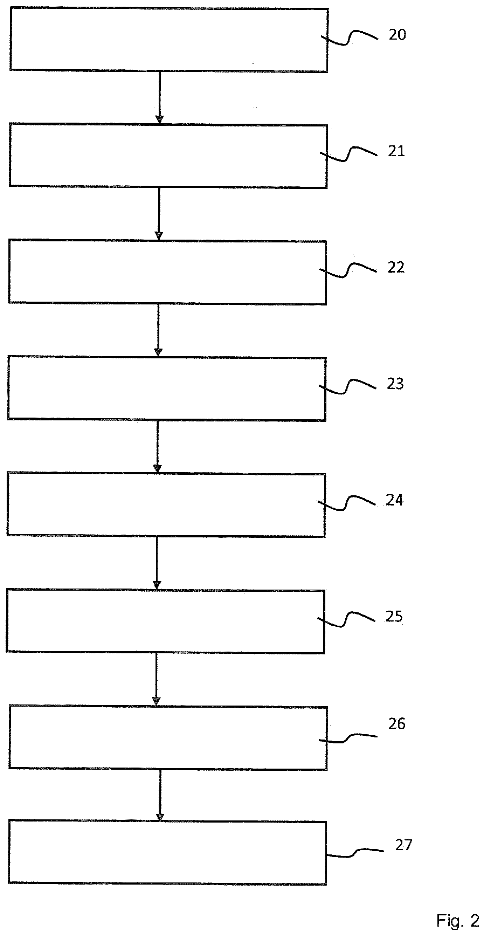

Mit Hilfe der in Fig. 1 gezeigten Vorrichtung ist ein Verfahren zum Bestimmen einer atypischen Bewegung einer Person, insbesondere zur Sturzerkennung, ausführbar. Fig. 2 zeigt eine schematische Blockdarstellung in Verbindung mit einer möglichen Ausführungsform eines solchen Verfahrens. Im Schritt 20 werden mit Hilfe der passiven IR-Detektionseinrichtung 2 IR-Signale für eine oder mehrere Personen in dem vorgelagerten Messraum erfasst. Die Nutzung des Thermopile-Arrays 3, wahlweise mehrerer Thermopile-Arrays, ermöglicht das Erfassen von Matrixsignalwert, das heißt einer zweidimensionalen Verteilung von IR-Messsignalen. Die IR-Messsignale werden digitalisiert. Das Thermopile-Array 3 kann beispielsweise eine Matrixfläche mit 8x8 Sensorpunkten aufweisen, die für die IR-Messung in dem Messraum einen Öffnungswinkel von beispielsweise 60 Grad ermöglichen. Es kann ein weiteres Thermopile-Array gleicher Ausbildung vorgesehen sein, so dass ein Öffnungswinkel von beispielsweise 120 Grad für die IR-Messung bei 16x8 Pixeln ermöglicht ist.

Der Benutzer kann über eine Konfigurationsoberfläche, zum Beispiel über eine von der Vorrichtung über WiFi übermittelte Website, wahlfrei Pixel aktiv lassen oder manuell deaktivieren, wenn diese nicht zur Auswertung verwendet werden sollen. Zum Beispiel wird in einem Schlafzimmer der Bereich des Betts als ungefährlich und relevant für den Schutz der Privatsphäre eingeschätzt und demnach aus dem Erfassungsbereich der Sensoranordnung entfernt.

Im Schritt 21 werden die IR-Messsignale mit Hilfe einer Filtereinrichtung (nicht dargestellt) gefiltert, um das Signal-Rausch-Verhältnis zu verbessern. Hierfür kann ein Tiefpass Anwendung finden. Alternativ oder ergänzend kann eine Mittelwertbildung ausgeführt werden.

Im Schritt 22 wird mit Hilfe der Auswerteeinrichtung 4 für die IR-Messsignale eine Objekterkennung und gegebenenfalls eine Segmentierung ausgeführt. Verfahren für derartige Datenbehandlungen sind als solche in verschiedenen Ausführungsformen bekannt. Hierbei sollen aus den IR-Messsignalen infrarotstrahlende Objekte (Zielperson) erkannt und vom Hintergrund isoliert werden. Mögliche Verfahren, die hierbei zum Einsatz kommen können, betreffen pixelorientierte, kantenbasierte und / oder regionenorientierte Verfahren sowie modellbasierte Verfahren. In einem Ausführungsbeispiel kann ein kantenbasiertes Verfahren unter Verwendung von Laplace-Operatoren zum Einsatz kommen. Die IR-Messsignale für die Pixel des Thermopile-Arrays 3 zeigen jeweils eine Pixeltemperatur an. Für den Übergang zwischen benachbarten Pixeln können hierbei die erste und zweite Ableitung gebildet werden und eine Nullstellenanalyse kann durchgeführt werden.

Im Schritt 23 wird ein Interpolationsverfahren ausgeführt. Der Schritt 23 kann auch weggelassen werden. Zur Vorbereitung einer Objektanalyse werden die IR-Messsignale interpoliert, zum Beispiel die Messsignale von 8x8 Pixeln. An sich bekannte bilineare und / oder bikubische Interpolationen können ausgeführt werden. Zum Einsatz kommen kann zum Beispiel eine Interpolation mittels normierter Distanzfunktionen.

Einige Schritte des Verfahrens sind in der Reihenfolge vertauschbar; insbesondere die Schritte 22 und 23.

Im Schritt 24 werden geometrische Daten der zuvor erkannten Objekte analysiert, zum Beispiel eine y-Ausdehnung, eine x-Ausdehnung, ein Temperaturschwerpunkt ("Center of Gravity"), eine Umrisslänge, eine Energiedichte, eine Energieverteilung und / oder Bewegungen der Obekte oder Ihrer jeweiligen Schwerpunkte. Die hierbei gewonnenen Merkmalsdaten werden aus den Sensordaten extrahiert. Die aus den Bilddaten gewonnenen erkannten Objekte werden binarisiert und zusammengefasst, beispielsweise nach dem bekannten Verfahren "Flood filling". Die objektbezogenen extrahierten Merkmalsdaten können nun weiter qualifiziert werden, zum Beispiel wie folgt: Person steht / liegt, Objekt bewegt sich, Objekt nähert sich dem Boden, Objekt bewegt sich nicht / bleibt liegen.

In einem separaten Schritt können diese segmentierten Objekte durch Expertenwissen noch weiter korrigiert werden, bzw. einige Besonderheiten kompensiert werden. Zum Beispiel sitzt ein Mensch dauerhaft auf einer Couch, steht dann auf und läuft durch den Raum. Durch die angewärmte Couch erkennt der Sensor einerseits das Wärmebild der aufstehenden Person, andererseits jedoch auch die Restwärme der Couch-Rückenlehne. Da die Person nicht plötzlich deutlich breiter werden kann, wird zum Beispiel die bewegte, neue und sich verändernde Kante des Umrisses als gültig angenommen und - unter Berücksichtigung einer konstanten Strahlungsenergie / Objektgröße) die "alte" stehende, ggfs. gegenüberliegende Kante als ungültig angesehen.

In einem weiteren Beispiel wird das Sensorbild einer Person durch ein Hindernis abgeschnitten oder gar aufgeteilt (beispielsweise Tischkante). Ein mögliches Vorgehen hier ist das gezielte Zusammensetzen der beiden entstehenden Teilobjekte zu einem gemeinsamen Objekt durch Lückenfüllung.

Mithilfe von Expertenwissen werden diese Objekte also korrigiert.

Neben den oben ausgeführten Merkmalen können auch genetische Algorithmen verwendet werden, um zunächst zufallsbasiert ausgewählte Merkmale der segmentierten Objekte auszuwählen (zum Beispiel Orientierungen der Objekte, Kantenlängen, Position der Objekte im Raum, Objektgeschwindigkeiten, Masseverteilungen etc.). Mithilfe vorher gewonnener Testdaten, die von Menschen als "Sturz" oder lein Sturz" qualifiziert wurden, ist es nun möglich, die Merkmale in Hinblick auf ihre Relevanz für eine Klassifizierung "Sturz" bzw. "kein Sturz" computergestützt untersuchen zu lassen und somit weitere relevante Merkmale aus den jeweiligen Objekten zu extrahieren und für eine Klassifizierung hinzuzuziehen.
Im Schritt 25 werden mögliche stationäre Störstrahlen unterdrückt, zum Beispiel elektrische Wärmequellen. Beispielsweise können sich nicht bewegende IR-strahlende Objekte als stationär und daher für das Erkennen der atypischen Bewegung als irrelevant eingestuft werden, insbesondere eine Heizung oder ein elektrisches Gerät ("Background Substraction").

Sodann werden im Schritt 26 die erkannten oder identifizierten Objekte gezählt. Wenn die Anzahl der erkannten beweglichen Objekte größer als 1 ist, kann davon ausgegangen werden, dass keine Gefahr besteht oder im Fall eines Sturzes Hilfe geholt werden kann. Eine Signalisierung etwaig erkannter Stürze kann dann unterdrückt werden.

Gemäß Schritt 27 werden die erkannten Objekte verfolgt und gepflegt. Hierbei können die extrahierten Merkmalsdaten aus dem Schritt 24 im Hinblick auf die Bewegung verfolgt werden. Bislang nicht unterdrückbare Rausch-Störeffekte können mit Hilfe dieser Informationen kompensiert werden. Beispielsweise wird ein Objekt, welches nur für sehr kurze Zeit erfasst wird, als Störeffekt / Rauschen verworfen. Ebenso werden bekannte Objekte, die für kurze Zeit nicht erfasst und dann wieder erfasst werden, als durchgängig vorhanden angenommen.

Schließlich wird im Schritt 27 eine Bewegungsanalyse für die erkannten oder identifizierten Objekte ausgeführt. Bewegungen in x-, y- sowie z-Richtung sind hierbei einerseits für die Pflege der erfassten objektbezogenen Daten wichtig, insbesondere die Objektzählung, und andererseits als Aktivitätsindex. Der Aktivitätsindex kann beispielsweise anzeigen, dass eine Person gesund ist und sich normal bewegt. Insbesondere abnorme Bewegungsmuster, zum Beispiel in negativer Y-Richtung, das heißt auf den Boden zu, können einen Sturz andeuten. In diesem Fall werden Geschwindigkeit, Beschleunigung und Position des Objekts analysiert. Diese elektronischen Informationen werden später im Hinblick auf die Erkennung eines möglichen Sturzes berücksichtigt. Mit Hilfe von sogenannten State-Machine-basierten Ansätzen und / oder Hidden-Markov-Modellen und / oder Support-Vektor-Maschinen können vorgegebene Muster einer atypischen Bewegung erkannt werden, beispielsweise eines Sturzes. Zur Auswertung kommen hierbei insbesondere die Anzahl der erkannten Objekte (Personen) wie auch die Reihenfolge der analysierten Daten. Die extrahierten Merkmalsdaten können in einer Liste aktualisiert und gepflegt werden. Historische Daten, aus denen sich eine Bewegung ergibt oder noch ergeben kann, können ebenfalls entsprechend gespeichert werden.

Ein Beispiel für das Erkennen einer atypischen Bewegung, insbesondere eines Sturzes, könnte daher Folgendes umfassen. Es wird ein einzelnes stehendes oder gehendes Objekt (Person, ohne helfende Person in der Nähe) detektiert. Die Bewegungsanalyse (Schritt 27) zeigt eine Bewegung nach unten in Richtung Fußboden an. Es ändert sich die Geometrie des erkannten Objekts, beispielsweise wird das Verhältnis der Ausmaße von Y zu X kleiner (Person steht nicht mehr). Auch findet keine Bewegung des erkannten Objektes mehr statt. Hierauf kann ein Sturz erkannt werden.

Die Vorrichtung aus Fig. 1 stellt im Fall des Erkennens der atypischen Bewegung Kommunikations- und Alarmierungsmöglichkeiten zur Verfügung. Mit Hilfe der Kommunikationseinrichtung 10 kann eine Telefonverbindung aufgebaut werden. Hierzu ist eine Mikrofon- und Lautsprecheranordnung vorgesehen, vergleichbar zum Beispiel mit einer Freisprecheinrichtung.

Mit Hilfe der Notfallsignaleinrichtung 14 kann eine Notfallnachricht abgesendet werden, zum Beispiel in Form einer SMS oder einer E-Mail.

Mittels der Signalisierungseinrichtung 8 kann die Notfallsituation signalisiert werden, mittels eines optischen und / oder eines akustischen Signals.

Alternativ oder ergänzend kann die Nachprüfeinrichtung 5 genutzt werden, um die Wahrscheinlichkeit für ein falsches Erkennen einer Notfallsituation zu mindern. Beim Erkennen einer kritischen Situation in Folge der Auswertung der IR-Messsignale kann unter Einbeziehung der Nachprüfeinrichtung 5 ein Telefonanruf initiiert werden, um beispielsweise mit einem Angehörigen Rücksprache zu halten. Alternativ oder ergänzend kann über die integrierte Lautsprechereinrichtung eine akustische Ausgabe (Ansprache) in den überwachten Messraum ausgegeben werden. Auch kann alternativ oder ergänzend eine optische Signalisierung erfolgen. Hierbei kann überwacht werden, ob auf die Signalisierung eine Rückmeldung von der überwachten Person detektiert wird, zum Beispiel ein Druck eines Tasters an der Vorrichtung, zum Beispiel auf der Frontplatte am Gehäuse 1, und / oder eine akustische Rückmeldung über die Mikrofoneinrichtung. Letztere kann einer Spracherkennung zugeführt werden. Wird ein als Sturz erkanntes Ereignis detektiert und der Benutzer quittiert es nicht zutreffend (Falscherkennung), kann das laufende Erkennungsverfahren unschärfer gestellt werden. Der Algorithmus wird also rekonfiguriert oder optimiert.

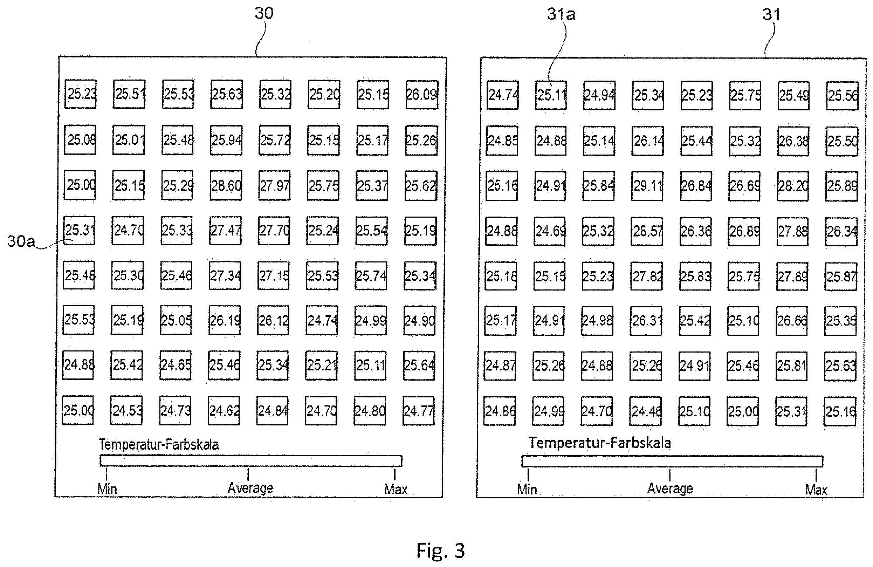

Fig. 3 zeigt eine schematische Darstellung von zwei Matrixanordnungen 30, 31 von Sensorpixeln 30a, 31a für Thermopile-Arrays, beispielsweise das Thermopile-Array 3.

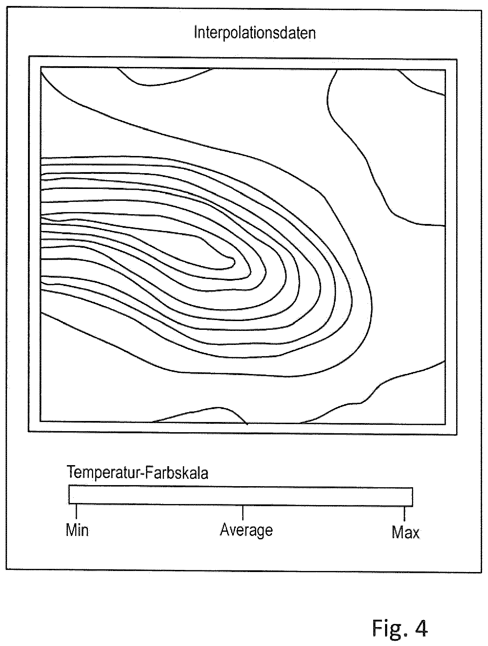

Fig. 4 zeigt eine schematische Darstellung von Interpolationsdaten.

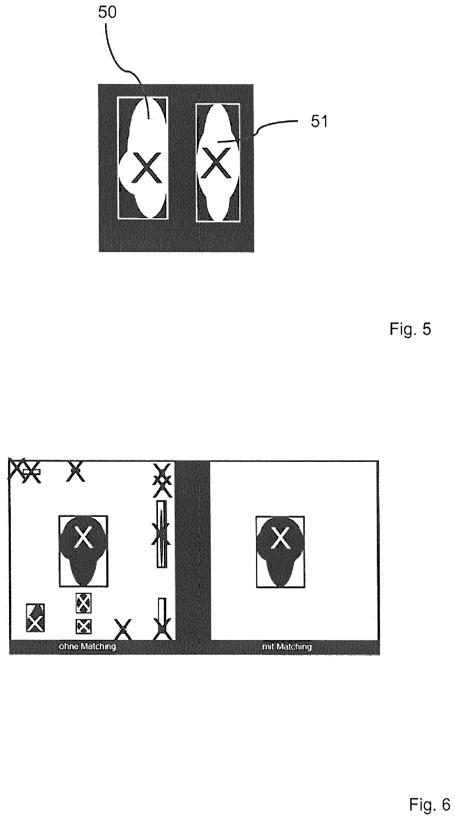

Fig. 5 zeigt eine schematische Darstellung für zwei erkannte Objekte 50, 51.

Fig. 6 zeigt eine schematische Darstellung in Verbindung mit der Filterung der IR-Messsignale vor (linke Seite) sowie nach dem Ausführen einer Rauschunterdrückung (rechte Seite).

Mit Hilfe der beschriebenen Technologien können bei den verschiedenen Ausgestaltungen ein oder mehrere der folgenden Vorteile erreicht werden. Lediglich eine passive Auswertung von vorhandenen Signalen aufgrund von Infrarotstrahlung vom Menschen kann vorgesehen sein. Ein berührungsloses Messen ist möglich. Eine einfache Installation ist gegeben, zum Beispiel lediglich ein Gerät pro Raum, was bauliche Veränderungen vermeidet. Es sind keine Tags oder mobile Sensoren am Körper des Nutzers notwendig. Die Verwendung von Batterien kann vermieden werden, was Service-Intervalle vermeidet.

Mehrere Möglichkeiten können zur Vermeidung von falschen Positiv-Meldungen (Erkennen von atypischer Bewegung) mittels Feedback vom Benutzer zum Einsatz kommen. Erfasste Bewegungsdaten können ausgewertet werden, zum Beispiel zurückgelegter Weg, Aktivität, um so ein medizinisch / therapeutisch hilfreiches Feedback zu geben. Aus datenschutzrechtlichen Gründen kann vorgesehen sein, hierfür das Einverständnis des Benutzers vorab, also vor dem Freischalten dieser Funktion, einzuholen.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Vorrichtung zum Bestimmen einer atypischen Bewegung einer Person, mit
- einem Gehäuse (1),
- einer passiven IR-Detektionseinrichtung (2) in dem Gehäuse (1), die ein Thermopile-Array (3) aufweist, bei dem eine flächige Sensoreinrichtung auf einer Sichtseite des Gehäuses (1) angeordnet ist, derart, dass IR-Strahlung von einer oder mehreren Personen in einem der Sichtseite vorgelagerten Messraum detektierbar ist, und
- einer Auswerteeinrichtung (4) in dem Gehäuse (1), die an die passive IR-Detektionseinrichtung (2) koppelt und eingerichtet ist, im Rahmen eines Prozesses zum Bestimmen einer atypischen Bewegung von der passiven IR-Detektionseinrichtung (2) als eine zweidimensionale Verteilung von IR-Messsignalen bereitgestellte IR-Detektorsignale, die die Anwesenheit einer detektierten Person in dem Messraum anzeigen, zu empfangen und auszuwerten, um eine atypische Bewegung der detektierten Person in dem Messraum zu bestimmen und ein das Bestimmen der atypischen Bewegung anzeigendes Signal an einem Datenausgang der Auswerteeinrichtung (4) bereitzustellen,
wobei die Auswerteeinrichtung (4) eingerichtet ist, zum Bestimmen der atypischen Bewegung, für die zweidimensionale Verteilung von IR-Messsignale,
- eine Objekterkennung auszuführen, um Objekte zu erkennen,
- für die erkannten Objekte geometrische Daten zu analysieren, um Merkmalsdaten zu extrahieren, wobei die geometrische Daten aus der folgenden Gruppe ausgewählt sind: eine y-Ausdehnung, eine x-Ausdehnung, ein Temperaturschwerpunkt, eine Umrisslänge, eine Energiedichte, eine Energieverteilung, Bewegungen der Objekte und ein jeweiliger Schwerpunkt der Objekte,
- die erkannten Objekte zu zählen, und
- für die erkannten Objekte eine Bewegungsanalyse für Bewegungen in x-, y- und z-Richtung auszuführen für eine Verfolgung und Pflege der objektbezogenen extrahierten Merkmalsdaten und als Aktivitätsindex.

2. Vorrichtung nach Anspruch 1, **gekennzeichnet durch** eine Nachprüfeinrichtung (5) in dem Gehäuse (1), die an die Auswerteeinrichtung (4) koppelt und eingerichtet ist, als Reaktion auf ein vorläufiges Erkennen der atypischen Bewegung beim Prozesses zum Bestimmen der atypischen Bewegung eine Nutzereingabe von der detektierten Person zu detektieren.

3. Vorrichtung nach Anspruch 2, **dadurch gekennzeichnet, dass** die Nachprüfeinrichtung (5) eingerichtet ist, ein optisches Nutzersignal, ein akustisches Nutzersignal und / oder ein Nutzerberührungssignal zu detektieren.

4. Vorrichtung nach mindestens einem der vorangehenden Ansprüche, **gekennzeichnet durch** eine Signalisierungseinrichtung (8) in dem Gehäuse (1), die an die Auswerteeinrichtung (4) koppelt und eingerichtet ist, auf das vorläufige Erkennen der atypischen Bewegung durch die Auswerteeinrichtung (4) ein optisches und / oder ein akustisches Signal auszugeben.

5. Vorrichtung nach mindestens einem der vorangehenden Ansprüche, **gekennzeichnet durch** eine elektronische Filtereinrichtung (12) in dem Gehäuse (1), die zumindest an die IR-Detektionseinrichtung (2) koppelt und eingerichtet ist, die von der passiven IR-Detektionseinrichtung (2) bereitgestellten Detektorsignale zu filtern.

6. Vorrichtung nach mindestens einem der vorangehenden Ansprüche, **gekennzeichnet durch** eine Kommunikationseinrichtung (10) in dem Gehäuse (1), die an die Auswerteeinrichtung (4) koppelt und eingerichtet ist, als Reaktion auf das Erkennen der atypischen Bewegung ein Kommunikationssignal abzusetzen.

7. Vorrichtung nach mindestens einem der vorangehenden Ansprüche, **gekennzeichnet durch** eine Notfallsignaleinrichtung (14) in dem Gehäuse (1), die an die Auswerteeinrichtung (4) koppelt und eingerichtet ist, als Reaktion auf das Erkennen der atypischen Bewegung ein Notfallsignal abzusetzen.

8. Vorrichtung nach mindestens einem der vorangehenden Ansprüche, **dadurch gekennzeichnet, dass** das Gehäuse (1) zumindest teilweise unter Verwendung einer Unterputz- oder einer Aufputz-Schalterdose gebildet ist.

9. Vorrichtung nach mindestens einem der vorangehenden Ansprüche, **dadurch gekennzeichnet, dass** die Sichtseite auf einer Schalterfrontplatte gebildet ist.

10. Vorrichtung nach mindestens einem der vorangehenden Ansprüche, **gekennzeichnet durch** eine Anschlusseinrichtung in dem Gehäuse (1), die an die passive IR-Detektionseinrichtung (2) und die Auswerteeinrichtung (4) koppelt und mit einem Spannungsversorgungsnetz verbunden und eingerichtet ist, die passive IR-Detektionseinrichtung (2) und die Auswerteeinrichtung (4) mit einer jeweiligen Betriebsspannung zu versorgen.

11. Verfahren zum Bestimmen einer atypischen Bewegung einer Person, bei dem
- mittels einer passiven IR-Detektionseinrichtung (2), die in einem Gehäuse (1) angeordnet ist und ein Thermopile-Array (3) mit einer flächigen Sensoreinrichtung auf einer Sichtseite des Gehäuses (1) aufweist, IR-Strahlung für eine oder mehrere Personen in einem der Sichtseite vorgelagerten Messraum detektiert wird, und
- mittels einer Auswerteeinrichtung (4), die in dem Gehäuse (1) angeordnet ist und an die passive IR-Detektionseinrichtung (2) koppelt, im Rahmen eines Prozesses zum Bestimmen einer atypischen Bewegung von der passiven IR-Detektionseinrichtung (2) als eine zweidimensionale Verteilung von IR-Messsignalen bereitgestellte IR-Detektorsignale, die die Anwesenheit einer detektierten Person in dem Messraum anzeigen, empfangen und ausgewertet werden, um eine atypische Bewegung der detektierten Person in dem Messraum zu bestimmen und ein das Bestimmen der atypischen Bewegung anzeigendes Signal an einem Datenausgang der Auswerteeinrichtung (4) bereitzustellen,
wobei mittels der Auswerteeinrichtung (4) zum Bestimmen der atypischen Bewegung, für die zweidimensionale Verteilung von IR-Messsignale,
- eine Objekterkennung ausgeführt wird, um Objekte zu erkennen,
- für die erkannten Objekte geometrische Daten analysiert werden, um Merkmalsdaten zu extrahieren, wobei die geometrische Daten aus der folgenden Gruppe ausgewählt sind: eine y-Ausdehnung, eine x-Ausdehnung, ein Temperaturschwerpunkt, eine Umrisslänge, eine Energiedichte, eine Energieverteilung, Bewegungen der Objekte und ein jeweiliger Schwerpunkt der Objekte,
- die erkannten Objekte gezählt werden, und
- für die erkannten Objekte eine Bewegungsanalyse für Bewegungen in x-, y- und z-Richtung ausgeführt wird für eine Verfolgung und Pflege der objektbezogenen extrahierten Merkmalsdaten und als Aktivitätsindex.

## Claims

1. Device for determining an atypical movement of a person, comprising
- a housing (1),
- a passive IR detection means (2) in the housing (1), which means has a thermopile array (3) in which a flat sensor means is arranged on a visible side of the housing (1) such that IR radiation from one or more people in a measurement room in front of the visible side can be detected, and
- an evaluation means (4) in the housing (1), which means is coupled to the passive IR detection means (2) and is configured, as part of a process for determining an atypical movement, to receive and evaluate IR detector signals which are provided by the passive IR detection means (2) as a two-dimensional distribution of IR measurement signals and which indicate the presence of a detected person in the measurement room in order to determine an atypical movement of the detected person in the measurement room and to provide a signal indicating the determination of the atypical movement at a data output of the evaluation means (4),
wherein the evaluation means (4) is configured, in order to determine the atypical movement, for the two-dimensional distribution of IR measurement signals,
- to carry out object identification in order to identify objects,
- to analyze geometric data for the identified objects in order to extract feature data, wherein the geometric data is selected from the following group: a y-dimension, an x-dimension, a temperature center of gravity, an outline length, an energy density, an energy distribution, movements of the objects and a center of gravity of each of the objects,
- to count the identified objects, and
- to carry out a movement analysis for movements in the x, y and z directions for the identified objects for tracking and maintaining the object-related extracted feature data and as an activity index.

2. Device according to claim 1, **characterized by** a verification means (5) in the housing (1), which means is coupled to the evaluation means (4) and is configured, as a response to a preliminary identification of the atypical movement in the process for determining the atypical movement, to detect a user input by the detected person.

3. Device according to claim 2, **characterized in that** the verification means (5) is configured to detect an optical user signal, an acoustic user signal and/or a user touch signal.

4. Device according to at least one of the preceding claims, **characterized by** a signaling means (8) in the housing (1), which means is coupled to the evaluation means (4) and is configured to output an optical and/or an acoustic signal upon preliminary identification of the atypical movement by the evaluation means (4).

5. Device according to at least one of the preceding claims, **characterized by** an electronic filter means (12) in the housing (1), which means is coupled at least to the IR detection means (2) and is configured to filter the detector signals provided by the passive IR detection means (2).

6. Device according to at least one of the preceding claims, **characterized by** a communication means (10) in the housing (1), which means is coupled to the evaluation means (4) and is configured to emit a communication signal in response to the identification of the atypical movement.

7. Device according to at least one of the preceding claims, **characterized by** an emergency signal means (14) in the housing (1), which means is coupled to the evaluation means (4) and is configured to emit an emergency signal in response to the identification of the atypical movement.

8. Device according to at least one of the preceding claims, **characterized in that** the housing (1) is at least partly formed using a flush-mounted or surface-mounted switch box.

9. Device according to at least one of the preceding claims, **characterized in that** the visible side is formed on a switch front panel.

10. Device according to at least one of the preceding claims, **characterized by** a connection means in the housing (1), which means is coupled to the passive IR detection means (2) and the evaluation means (4), is connected to a power supply network and is configured to supply the passive IR detection means (2) and the evaluation means (4) with operating voltage in each case.

11. Method for determining an atypical movement of a person, in which
- by means of a passive IR detection means (2) which is arranged in a housing (1) and has a thermopile array (3) having a flat sensor means on a visible side of the housing (1), IR radiation is detected for one or more people in a measurement room in front of the visible side, and
- by means of an evaluation means (4) which is arranged in the housing (1) and is coupled to the passive IR detection means (2), as part of a process for determining an atypical movement, IR detector signals which are provided by the passive IR detection means (2) as a two-dimensional distribution of IR measurement signals and which indicate the presence of a detected person in the measurement room are received and evaluated in order to determine an atypical movement of the detected person in the measurement room and to provide a signal indicating the determination of the atypical movement at a data output of the evaluation means (4),
wherein by means of the evaluation means (4), in order to determine the atypical movement, for the two-dimensional distribution of IR measurement signals,
- object identification is carried out in order to identify objects,
- geometric data for the identified objects is analyzed in order to extract feature data, wherein the geometric data is selected from the following group: a y-dimension, an x-dimension, a temperature center of gravity, an outline length, an energy density, an energy distribution, movements of the objects and a center of gravity of each of the objects,
- the identified objects are counted, and
- a movement analysis for movements in the x, y and z directions is carried out for the identified objects for tracking and maintaining the object-related extracted feature data and as an activity index.

## Revendications

1. Dispositif de détermination d'un mouvement atypique d'une personne, comportant
- un boîtier (1),
- un système de détection infrarouge passif (2) dans le boîtier (1), lequel système de détection infrarouge passif présente un réseau de thermopiles (3), dans lequel dispositif de détermination un système capteur plat est disposé sur un côté visible du boîtier (1) de telle sorte que le rayonnement infrarouge d'une ou plusieurs personnes peut être détecté dans un espace de mesure placé devant le côté visible, et
- un système d'évaluation (4) dans le boîtier (1), lequel système d'évaluation est accouplé au système de détection infrarouge passif (2) et est configuré, dans le cadre d'un processus de détermination d'un mouvement atypique du système de détection infrarouge passif (2), pour recevoir et évaluer en tant que distribution bidimensionnelle des signaux de détecteur infrarouge fournis par les signaux de mesure infrarouge, lesquels signaux de détecteur infrarouge indiquent la présence d'une personne détectée dans l'espace de mesure, afin de déterminer un mouvement atypique de la personne détectée dans l'espace de mesure et afin de fournir un signal indiquant la détermination du mouvement atypique à une sortie de données du système d'évaluation (4),
le système d'évaluation (4) étant configuré pour la détermination du mouvement atypique en vue de la distribution bidimensionnelle des signaux de mesure infrarouges,
- pour effectuer une reconnaissance d'objets afin de reconnaître des objets,
- pour analyser des données géométriques des objets reconnus afin d'extraire des données caractéristiques, les données géométriques étant sélectionnées dans le groupe suivant : une étendue y, une étendue x, un centre de température, une longueur de contour, une densité d'énergie, une distribution d'énergie, les mouvements des objets et un centre respectif des objets,
- pour compter les objets reconnus, et
- pour effectuer une analyse de mouvement pour les mouvements dans les directions x, y et z pour les objets reconnus afin de réaliser le suivi et la maintenance des données caractéristiques extraites liées aux objets et en tant qu'indice d'activité.

2. Dispositif selon la revendication 1, **caractérisé par** un système de contrôle (5) dans le boîtier (1), lequel système de contrôle est accouplé au système d'évaluation (4) et est configuré pour détecter une entrée utilisateur de la personne détectée en réponse à une reconnaissance préalable du mouvement atypique lors du processus de détermination du mouvement atypique.

3. Dispositif selon la revendication 2, **caractérisé en ce que** le système de contrôle (5) est configuré pour détecter un signal utilisateur optique, un signal utilisateur acoustique et/ou un signal utilisateur tactile.

4. Dispositif selon au moins l'une des revendications précédentes, **caractérisé par** un système de signalisation (8) dans le boîtier (1), lequel système de signalisation est accouplé au système d'évaluation (4) et configuré pour émettre un signal optique et/ou acoustique lors de la reconnaissance préalable du mouvement atypique par le système d'évaluation (4).

5. Dispositif selon au moins l'une des revendications précédentes, **caractérisé par** un système de filtrage électronique (12) dans le boîtier (1), lequel système de filtrage électronique est accouplé au moins au système de détection infrarouge (2) et est configuré pour filtrer les signaux de détecteur fournis par le système de détection infrarouge passif (2).

6. Dispositif selon au moins l'une des revendications précédentes, **caractérisé par** un système de communication (10) dans le boîtier (1), lequel système de communication est accouplé au système d'évaluation (4) et est configuré pour envoyer un signal de communication en réponse à la reconnaissance du mouvement atypique.

7. Dispositif selon au moins l'une des revendications précédentes, **caractérisé par** un système de signalisation de secours (14) dans le boîtier (1), lequel système de signalisation de secours est accouplé au système d'évaluation (4) et est configuré pour envoyer un signal de secours en réponse à la reconnaissance du mouvement atypique.

8. Dispositif selon au moins l'une des revendications précédentes, **caractérisé en ce que** le boîtier (1) est formé au moins partiellement en utilisant une prise de commutation encastrée ou en saillie.

9. Dispositif selon au moins l'une des revendications précédentes, **caractérisé en ce que** le côté visible est formé sur une plaque frontale de commutation.

10. Dispositif selon au moins l'une des revendications précédentes, **caractérisé par** un système de raccord dans le boîtier (1), lequel système de raccord est accouplé au système de détection infrarouge passif (2) et au système d'évaluation (4) et est relié à un réseau d'alimentation en tension, et est configuré pour alimenter le système de détection infrarouge passif (2) et le système d'évaluation (4) en une tension de fonctionnement respective.

11. Procédé de détermination d'un mouvement atypique d'une personne, dans lequel
- au moyen d'un système de détection infrarouge passif (2) qui est disposé dans un boîtier (1) et qui présente un réseau de thermopiles (3) comportant un système de capteur plat sur un côté visible du boîtier (1), un rayonnement IR pour une ou plusieurs personnes est détecté dans un espace de mesure placé devant le côté visible, et
- au moyen d'un système d'évaluation (4) qui est disposé dans le boîtier (1) et est accouplé au système de détection infrarouge passif (2) dans le cadre d'un processus de détermination d'un mouvement atypique du système de détection infrarouge passif (2), pour recevoir et évaluer en tant que distribution bidimensionnelle des signaux de détecteur infrarouge fournis par les signaux de mesure infrarouge, lesquels signaux de détecteur infrarouge indiquent la présence d'une personne détectée dans l'espace de mesure, afin de déterminer un mouvement atypique de la personne détectée dans l'espace de mesure et afin de fournir un signal indiquant la détermination du mouvement atypique à une sortie de données du système d'évaluation (4),
au moyen du système d'évaluation (4) pour la détermination du mouvement atypique en vue de la distribution bidimensionnelle des signaux de mesure infrarouges,
- une reconnaissance d'objets est effectuée afin de reconnaître des objets,
- des données géométriques sont analysées pour les objets reconnus afin d'extraire des données caractéristiques, les données géométriques étant sélectionnées dans le groupe suivant : une étendue y, une étendue x, un centre de température, une longueur de contour, une densité d'énergie, une distribution d'énergie, les mouvements des objets et un centre respectif des objets,
- les objets reconnus sont comptés, et
- une analyse de mouvement pour les mouvements dans les directions x, y et z est effectuée pour les objets reconnus afin de réaliser le suivi et la maintenance des données caractéristiques extraites liées aux objets et en tant qu'indice d'activité.
